# EUROPEAN PATENT APPLICATION

(11) **EP 4 223 239 A1**
(43) Date of publication of application: **09.08.2023**
(21) Application number: 21874400.1
(22) Date of filing: 26.09.2021
(51) Int. Cl.: A61B 17/34, A61B 8/00

(54) **ULTRASONOGRAPHIC DEVICE**

(30) Priority: 29.09.2020 CN 202011056010
(71) Applicant: Ultrasound Assisted Medtech Pte. Ltd., Shenzhen, Guangdong 518118 (CN)
(72) Inventor: YUAN, Jinqiang, Shenzhen, Guangdong 518118 (CN); ZHAO, Kena, Shenzhen, Guangdong 518118 (CN); MAO, Jiawei, Shenzhen, Guangdong 518118 (CN); ZHOU, Jia, Shenzhen, Guangdong 518118 (CN)
(74) Representative: Cabinet Laurent & Charras
(86) International application number: PCT/CN2021/120762
(87) International publication number: WO 2022/068740

(57) **Abstract**

The technical field of medical devices, in particular, an ultrasonographic device, is related. The ultrasonographic device includes a mounting housing (A1), an ultrasonic transducer (A2), and a puncture support (A700). The ultrasonic transducer (A2) is rotatably disposed in an inner cavity of the mounting housing (A1) and spaced apart from the mounting housing (A1). The puncture support (A700) is provided on an outer side of the mounting housing (A1), where a puncture guide hole (A721) through which a puncture needle (A900) penetrates is provided on the puncture support (A700), and the puncture support (A700) is capable of rotating synchronously with the ultrasonic transducer (A2) so that an axis of the puncture guide hole (A721) is coplanar with a real-time scanning plane of the ultrasonic transducer (A2).

## Description

The present application claims priority to Chinese Patent Application No. 202011056010.9 filed with the China National Intellectual Property Administration (CNIPA) on Sep. 29, 2020, the disclosure of which is incorporated herein by reference in its entirety.

### TECHNICAL FIELD

The present application relates to the technical field of medical devices, for example, an ultrasonographic device.

### BACKGROUND

Ultrasonography is the basic means for a doctor to obtain lesion information and also the key to diagnose diseases. An ultrasonic interventional diagnostic and therapeutic device generally includes an ultrasound probe and a puncture support mounted on the ultrasound probe. After the ultrasound probe diagnoses a lesion site, a puncture needle punctures at the lesion site, so as to obtain living tissue.

### SUMMARY

Embodiments of the present application provide an ultrasonographic device capable of achieving accurate puncture and obtaining a more accurate diagnosis and treatment effect.

Embodiments of the present application provide an ultrasonographic device. The ultrasonographic device includes a mounting housing, an ultrasonic transducer, and a puncture support. The ultrasonic transducer is rotatably disposed in an inner cavity of the mounting housing and spaced apart from the mounting housing. The puncture support is provided on an outer side of the mounting housing, where a puncture guide hole through which a puncture needle penetrates is provided on the puncture support, and the puncture support is capable of rotating synchronously with the ultrasonic transducer so that an axis of the puncture guide hole is coplanar with a real-time scanning plane of the ultrasonic transducer.

### BRIEF DESCRIPTION OF DRAWINGS

FIG. 1 is a structure view of an ultrasonographic device according to embodiment one of the present application;
FIG. 2 is a structure view of an ultrasound probe and a puncture support according to embodiment one of the present application;
FIG. 3 is a structure view of the ultrasound probe according to embodiment one of the present application;
FIG. 4 is a first partial structure view of the ultrasound probe and the puncture support according to embodiment one of the present application;
FIG. 5 is a first axonometric view of the puncture support according to embodiment one of the present application;
FIG. 6 is a second axonometric view of the puncture support according to embodiment one of the present application;
FIG. 7 is a second partial structure view of the ultrasound probe and the puncture support according to embodiment one of the present application;
FIG. 8 is a working schematic diagram of the ultrasound probe and the puncture support according to embodiment one of the present application;
FIG. 9 is a first partial structure view of an ultrasound probe and a puncture support according to embodiment two of the present application;
FIG. 10 is a second partial structure view of an ultrasound probe and a puncture support according to embodiment two of the present application;
FIG. 11 is a structure view of an ultrasound probe and a puncture support according to embodiment three of the present application;
FIG. 12 is a partial structure view of the ultrasound probe and the puncture support according to embodiment three of the present application;
FIG. 13 is a structure view of an ultrasound probe and a puncture support according to embodiment four of the present application;
FIG. 14 is a partial structure view of the ultrasound probe and the puncture support according to embodiment four of the present application;
FIG. 15 is a partial structure view of an ultrasound probe and a puncture support according to embodiment five of the present application;
FIG. 16 is a structure view of an ultrasound probe and a puncture support according to embodiment six of the present application;
FIG. 17 is a partial structure view of the ultrasound probe and the puncture support according to embodiment six of the present application;
FIG. 18 is a structure view of an ultrasound probe and a puncture support according to embodiment seven of the present application;
FIG. 19 is a partial structure view of the ultrasound probe and the puncture support according to embodiment seven of the present application;
FIG. 20 is a structure view of an ultrasound probe and a puncture support according to embodiment eight of the present application;
FIG. 21 is a partial structure view of the ultrasound probe and the puncture support according to embodiment eight of the present application;
FIG. 22 is a first exploded view of the puncture support according to embodiment eight of the present application;
FIG. 23 is a second exploded view of the puncture support according to embodiment eight of the present application;
FIG. 24 is a structure view of the puncture support in a downward oblique puncture state according to embodiment eight of the present application;
FIG. 25 is a structure view of a puncture support in a horizontal puncture state according to embodiment eight of the present application;
FIG. 26 is a structure view of a puncture support in an upward oblique puncture state according to embodiment eight of the present application;
FIG. 27 is a structure view of an ultrasonographic device in a horizontal puncture state according to embodiment nine of the present application;
FIG. 28 is an internal structure view of an ultrasonographic device according to embodiment nine of the present application;
FIG. 29 is a structure view of a housing according to embodiment nine of the present application;
FIG. 30 is a structure view of a puncture support according to embodiment nine of the present application;
FIG. 31 is a structure view of the ultrasonographic device in upward and downward puncture states according to embodiment nine of the present application;
FIG. 32 is an ultrasonic display diagram in a horizontal puncture state according to embodiment nine of the present application;
FIG. 33 is an ultrasonic display diagram in an upward puncture state according to embodiment nine of the present application; and
FIG. 34 is an ultrasonic display diagram in a downward puncture state according to embodiment nine of the present application.

### Reference list

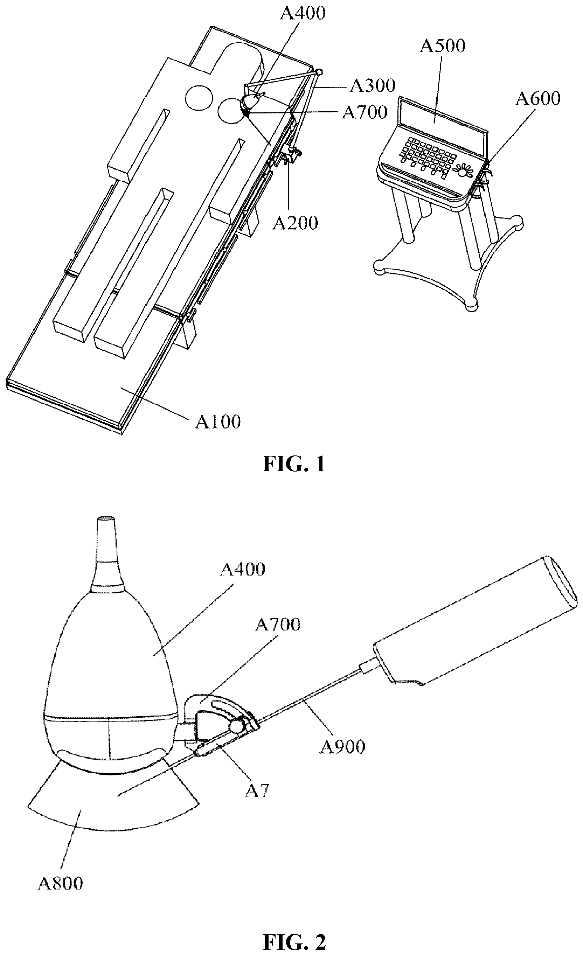

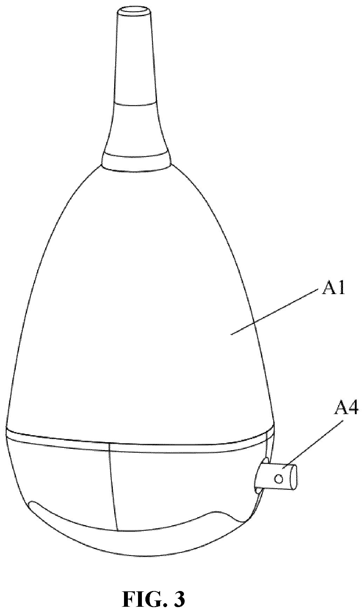

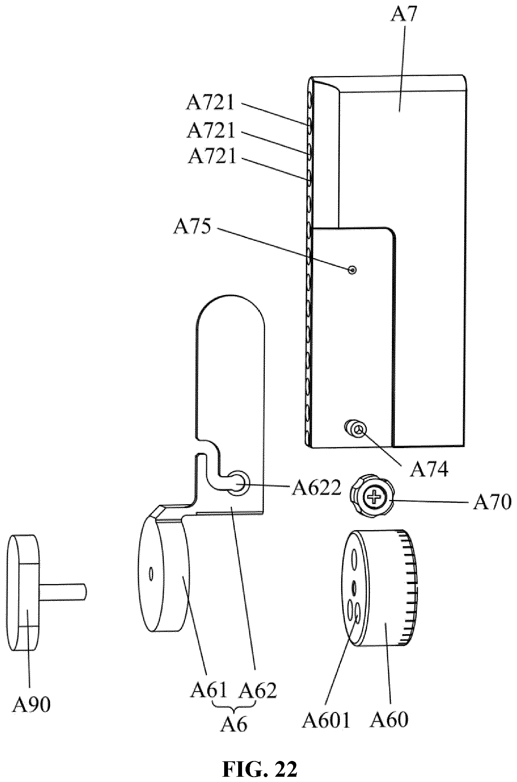

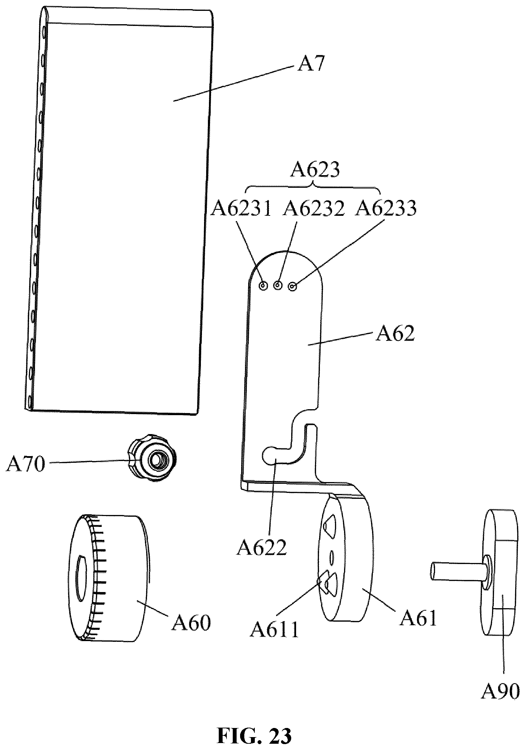

In FIGS. 1 to 26:
- A100: operating table
- A200: fixture
- A300: free arm clamping device
- A400: ultrasound probe
- A500: display
- A600: host
- A700: puncture support
- A800: scanning plane
- A900: puncture needle
- A1000: breast
- A1: mounting housing
- A2: ultrasonic transducer
- A3: first driver
- A4: first main shaft
- A5: first transmission mechanism
- A6: carrier
- A7: puncture guide member
- A8: second driver
- A9: second transmission mechanism
- A10: second main shaft
- A30: inner gear
- A20: outer gear
- A40: rack
- A50: gear
- A60: connecting stub shaft
- A601: first positioning groove
- A70: nut
- A80: connector (for example, a connection block)
- A90: fastener
- A11: probe end housing
- A12: housing
- A61: connecting portion
- A62: carrier body
- A611: first positioning protrusion
- A621: positioning groove
- A622: connecting hole
- A623: second positioning groove
- A6231: second positioning groove one
- A6232: second positioning groove two
- A6233: second positioning groove three
- A71: hinge bracket
- A72: guide member
- A73: protrusion
- A74: connecting post
- A75: second positioning protrusion
- A721: puncture guide hole

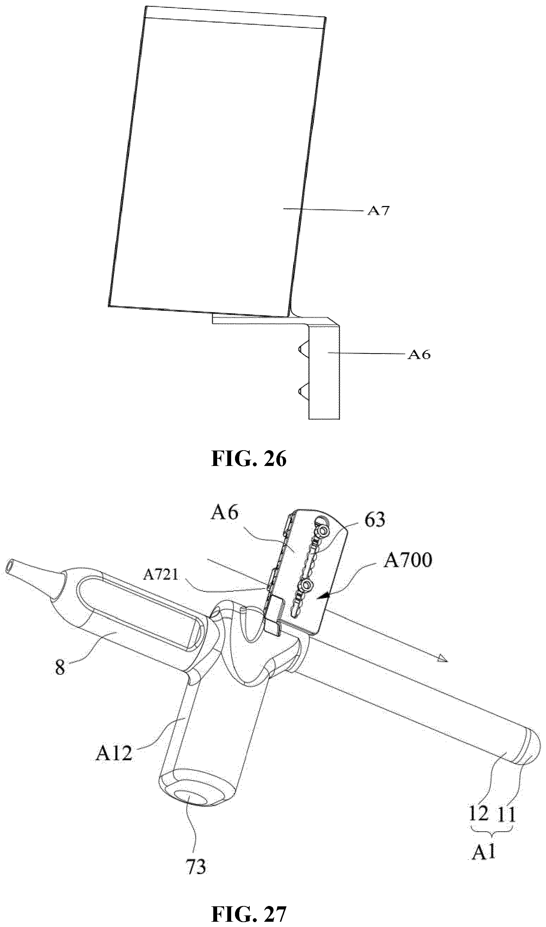

In FIGS. 27 to 34:
- A11: probe end housing
- A4: first main shaft
- A2: ultrasonic transducer
- A3: first driver
- 5: mounting ring
- A700: puncture support
- A12: housing
- 8: grip portion
- 9: mounting seat
- 11: head
- 12: acoustically transparent housing (for example, a hollow rod; for another example, an acoustically transparent hollow rod)
- A6: carrier
- A7: puncture guide member
- 63: adjustment slot
- 71: first opening
- 72: second opening
- 73: third opening
- 100: target object
- 200: horizontal puncture channel
- 300: upward oblique puncture channel
- 400: downward oblique puncture channel

### DETAILED DESCRIPTION

Taking a biopsy puncture operation of breast cancer as an example, a two-dimensional ultrasound probe equipped with a puncture support is generally used as a diagnostic device. However, it is relatively difficult for such diagnostic device to accurately find a puncture location and puncture at a designated location with a puncture needle to obtain living tissue. The breast is easily deformed under the rotational pressing of the ultrasound probe, it is difficult to accurately find and locate the breast cancer lesion by the two-dimensional ultrasound, and the staff needs to operate the device by hand. Therefore, it is difficult to ensure the operating stability. Finally, the puncture is very easy to deviate from the lesion, causing a secondary injury to a patient.

If a 3-dimension (3D) ultrasound probe equipped with a puncture support is used as the diagnostic device, an ultrasonic transducer is rotatably mounted inside a probe end housing, and the puncture support is fixed on the probe end housing. When the ultrasonic transducer rotates inside the probe end housing to achieve 3D imaging, the puncture support fixed on the probe end housing cannot rotate synchronously with the ultrasonic transducer, which makes it difficult to ensure that a puncture path of the puncture needle is on a scanning plane of the ultrasound probe and to achieve accurate puncture. Therefore, very few 3D ultrasound probes are used in ultrasonic interventional therapies. Most ultrasonic interventional therapies still use the two-dimensional ultrasound probe. However, the two-dimensional ultrasound probe can provide only two-dimensional ultrasound image information, resulting in great limitations for lesion diagnosis and puncture operation guidance.

In response to the preceding case, embodiments of the present application disclose an ultrasonographic device.

In the description of the present application, orientations or positional relationships indicated by terms such as "center", "up", "down", "left", "right", "vertical", "horizontal", "in", and "out" are based on orientations or positional relationships shown in FIGS. 1 to 34. These orientations or positional relationships are intended to facilitate the description of the present application and not to indicate or imply that a device or element referred to must have such particular orientations or be configured or operated in such particular orientations. In addition, terms such as "first" and "second" are used only for the description of features and are not to be construed as indicating or implying relative importance. Terms "first position" and "second position" are two different positions.

In the description of the present application, unless otherwise expressly specified and limited, the term "mounting", "connected to each other", or "connected" is to be construed in a broad sense, for example, as securely connected or detachably connected; mechanically connected or electrically connected; directly connected to each other or indirectly connected to each other via an intermediary; or internally connected between two elements.

### Embodiment one

As shown in FIGS. 1 to 6, this embodiment provides an ultrasonographic device. The ultrasonographic device includes a 3D ultrasound probe A400 and a puncture support A700 mounted on the ultrasound probe A400. The ultrasound probe A400 includes a mounting housing A1 and an ultrasonic transducer A2 disposed in the mounting housing A1. The ultrasonic transducer A2 is spaced apart from an inner wall of the mounting housing A1. The ultrasonic transducer A2 is rotatable in the mounting housing A1 to sweep the relevant site to be examined. The puncture support A700 is disposed outside the mounting housing A1. A puncture guide hole A721 through which a puncture needle A900 penetrates is provided on the puncture support A700. After the ultrasonic transducer A2 finds out the lesion, the puncture needle A900 is inserted into the lesion to obtain the living tissue.

The ultrasonographic device further includes an operating table A100 and a drive mechanism provided on the operating table A100. The patient lies on the operating table A100. The drive mechanism includes a fixture A200 and a free arm clamping device A300. The fixture A200 is fixed on the operating table A100. An output end of the fixture A200 is connected to the free arm clamping device A300, and the free arm clamping device A300 is connected to the mounting housing A1 of the ultrasound probe A400. The free arm clamping device A300 can be freely released, allowing a doctor to move the whole ultrasound probe A400 to a site to be examined of the patient and then lock the free arm clamping device A300.

The ultrasonographic device further includes a host A600 and a display A500. The display A500 is configured to display an ultrasound image formed after the ultrasound probe A400 scans the site to be examined. The host A600 controls the ultrasound probe A400, the puncture support A700, the drive mechanism, and the display A500 separately to work.

In this embodiment, in FIGS. 2, 5, and 7, the puncture support A700 is rotatable synchronously with the ultrasonic transducer A2 so that an axis of the puncture guide hole A721 on the puncture support A700 is always on a real-time scanning plane A800 of the ultrasonic transducer A2, so as to achieve accurate puncture and obtain more efficient and accurate diagnosis and treatment.

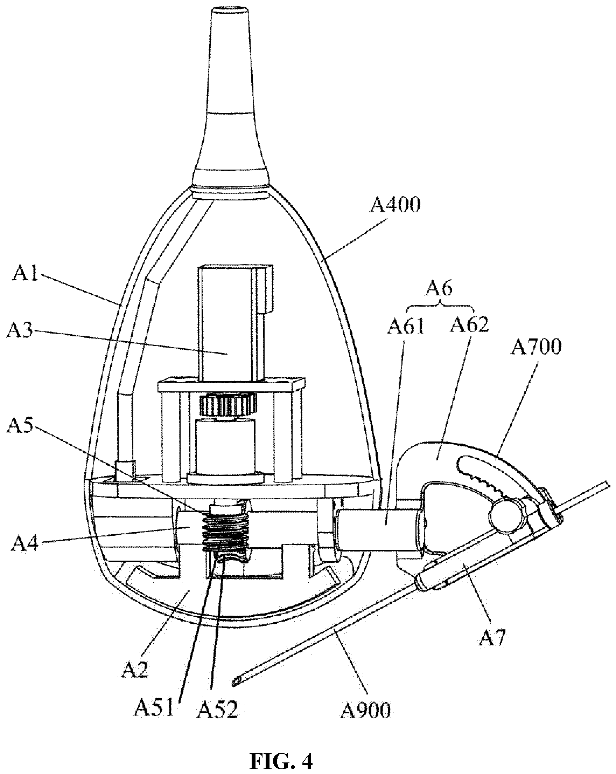

For example, in FIG. 4, the ultrasonographic device further includes a first driver A3 and a first main shaft A4 that are disposed in the mounting housing A1, where an output end of the first driver A3 is drivingly connected to the first main shaft A4 through a first transmission mechanism A5, and the first main shaft A4 is connected to the ultrasonic transducer A2. An avoiding hole is provided on the mounting housing A1, and the first main shaft A4 extends out of the mounting housing A1 through the avoiding hole and is connected to the puncture support A700.

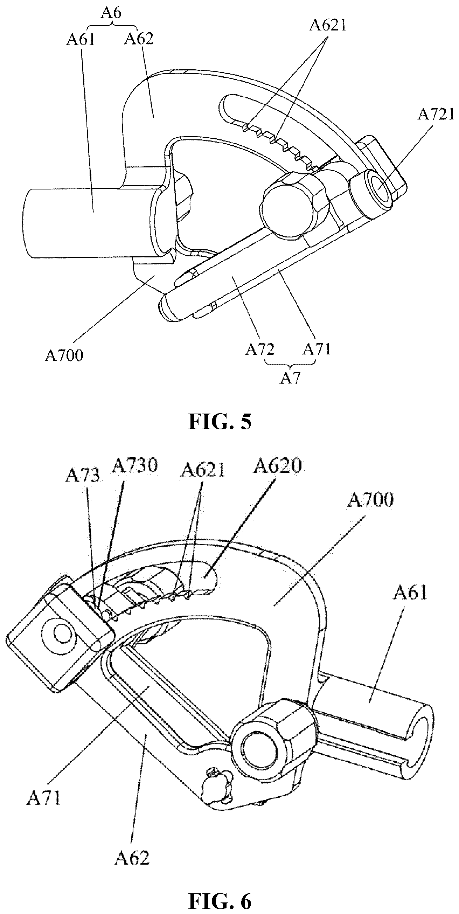

In FIG. 5, the first driver A3 drives, through the first transmission mechanism A5, the first main shaft A4 to rotate, and the first main shaft A4 drives the ultrasonic transducer A2 and the puncture support A700 to rotate synchronously so that the axis of the puncture guide hole A721 on the puncture support A700 is always on the real-time scanning plane A800 of the ultrasonic transducer A2. The first driver A3 may be an electric motor. Synchronous rotation of the ultrasonic transducer A2 with the puncture support A700 is achieved.

In an embodiment, the first transmission mechanism A5 is connected to the output end of the first driver A3 through a gear. The first transmission mechanism A5 includes a worm A51, a worm gear A52 is coaxially mounted on the first main shaft A4, and the worm A51 drives the worm gear A52 to rotate. The transmission between the worm gear and the worm with a self-locking function is adopted, without introducing electric motor torque locking or additional electric or manual locking. In other embodiments, the first transmission mechanism A5 may be drivingly connected to the first main shaft A4 through a belt drive, a chain drive, or the like.

In an embodiment, in FIGS. 2 and 4, the puncture support A700 includes a carrier A6 and a puncture guide member A7. The carrier A6 is fixedly connected to an end of the first main shaft A4 extending out of the mounting housing A1. The puncture guide member A7 is rotatably mounted on the carrier A6.

In FIGS. 4 to 6, the carrier A6 includes a connecting portion A61 and a carrier body A62 that are connected to each other. The connecting portion A61 may be tubular or semi-tubular, and the connecting portion A61 is sleeved at the end of the first main shaft A4 extending out of the mounting housing A1. The connecting portion A61 is fixed to the first main shaft A4 by a fastener, a clip, or the like. For example, the connecting portion A61 partially extends into the mounting housing A1 through the avoiding hole, so as to prevent impurities such as external dust or water vapor from entering the inside of the mounting housing A1 through a gap between the first main shaft A4 and a hole wall of the avoiding hole, thereby achieving sealing and ensuring a service life of the ultrasonic transducer A2. A sealing ring may be sleeved on an outer wall of the connecting portion A61 to improve the sealing property.

In an embodiment, in FIGS. 5 and 6, the puncture guide member A7 includes a hinge bracket A71 and a guide member A72. The puncture guide hole A721 is provided on the guide member A72. The guide member A72 is fixed to the hinge bracket A71. The bottom of the hinge bracket A71 (an end facing the patient) is hinged to the carrier body A62. A chute A620 is provided on the carrier body A62. A mating portion A730 is provided at the top of the hinge bracket A71 (an end facing away from the patient) and clamped into the chute A620. The mating portion A730 is slidable in the chute A620. The mating portion A730 and the chute A620 can be fixed relative to each other through a fastener.

In an embodiment, multiple positioning grooves A621 are provided on a groove wall of the chute A620. A protrusion A73 is provided on the mating portion A730. The hinge bracket A71 is rotated so that the protrusion A73 can be clamped into any positioning groove A621, so as to achieve the angular adjustment of the whole puncture guide member A7 along the carrier body A6 in the scanning plane A800 of the ultrasonic transducer A2, thereby ensuring that the puncture needle A900 is accurately inserted into the lesion. Each positioning groove A621 corresponds to a number and different positioning grooves A621 correspond to different numbers. The protrusion A73 is clamped into different positioning grooves A621, corresponding to a different angle of rotation of the puncture guide member A7.

In addition, multiple puncture guide holes A721 with different dimensions may be provided on the guide member A72 to adapt to puncture needles A900 with different dimensions, enabling adaptation to puncture at different sites.

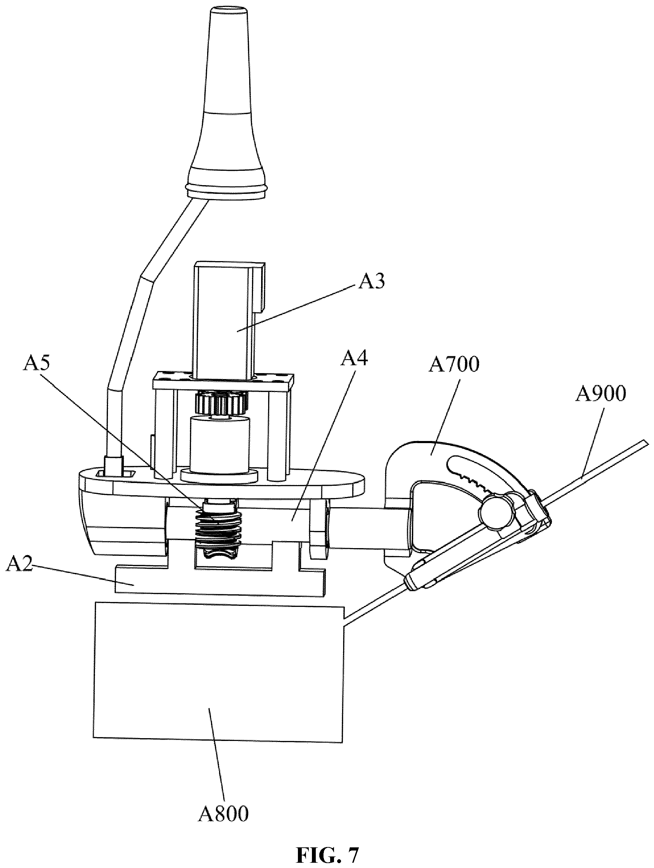

The ultrasonic transducer A2 in FIG. 4 is a convex array ultrasonic transducer with good imaging quality and can be used for detection of deeper sites in the body. The ultrasonic transducer A2 shown in FIG. 7 is a linear array ultrasonic transducer that has better image quality of superficial tissue and can provide better ultrasonic interventional diagnosis and treatment for the superficial tissue.

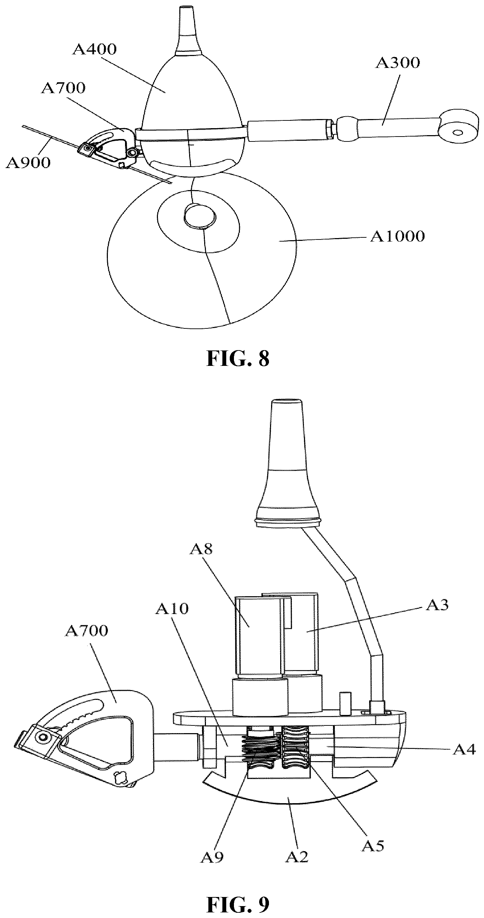

Referring to FIG. 8, a method for using the ultrasonographic device provided in this embodiment is generally described below.

Taking the lesion puncture of a breast A1000 as an example, the mounting housing A1 of the ultrasound probe A400 is pressed on a surface of the breast, and the mounting housing A1 remains stationary, avoiding the tissue deformation of the breast caused by the movement of the mounting housing A1 and ensuring the accuracy of subsequent ultrasound images. In FIG. 7, the first driver A3 drives the first main shaft A4 to rotate through the first transmission mechanism A5, and the first main shaft A4 drives the ultrasonic transducer A2 to rotate, so as to scan and form a 3D ultrasound image of part of the breast and display the 3D ultrasound image on the display A500. In FIGS. 5 and 6, after a puncture target is selected in the ultrasound image, a system automatically rotates the ultrasonic transducer A2 and the puncture support A700 to a plane where the puncture target is located and gives the number of the positioning groove A621, and the puncture guide member A7 is manually rotated so that the protrusion A73 is clamped into the positioning groove A621 of the corresponding number, and the puncture guide member A7 is aligned with the puncture target. The puncture needle A900 is inserted into the puncture guide hole A721 of the puncture guide member A7 and inserted into the breast along a preset path. In the insertion process, whether the puncture needle A900 reaches a preset position according to a preset trajectory is determined by comparing an actual trajectory of the puncture needle A900 in the ultrasound image with a theoretical trajectory of the puncture needle A900. The puncture is accurate and the puncture efficiency is high.

Since the puncture needle A900 is relatively thin, the puncture needle A900 may be bent or deflected when inserted into the tissue. In consideration of patient safety, monitoring of an actual position of the puncture needle A900 is extremely important. If the puncture needle A900 bends in an image plane, the bending deformation can be monitored in the image plane. As to the case where the puncture needle A900 bends outside the image plane, it can be determined based on whether the puncture needle A900 can be seen in the image. The bending deformation of the puncture needle A900 in the image plane may be corrected by fine-tuning the puncture guide member A7. If the puncture needle A900 bends outside the image plane, correction must be performed by pulling out the puncture needle A900 and inserting the puncture needle A900 again.

In addition to the puncture of the breast A1000, the ultrasonographic device may also be used in operations such as radioactive particle implantation, cryoneedle therapy, and radiofrequency ablation needle therapy and can provide the accuracy required for targeted therapy.

In the ultrasonographic device provided in this embodiment, the 3D ultrasound probe A400 can provide the 3D ultrasound image, and the puncture support A700 rotates synchronously with the ultrasonic transducer A2, so as to ensure that the puncture path is always within the real-time scanning plane A800 of the ultrasonic transducer A2 (that is, the axis of the puncture guide hole A721 of the puncture guide member A7 (i.e., the puncture path) is always within the real-time scanning plane A800 of the ultrasonic transducer A2), and at the same time, a position of the puncture guide member A7 on the carrier A6 can be manually adjusted, so as to provide accuracy positioning for the puncture operation, improve the puncture efficiency and accuracy, reduce the operation time, reduce the operation difficulty, and achieve accurate, low-invasive, simple, and quick surgical treatment.

### Embodiment two

The difference between this embodiment and embodiment one is described below.

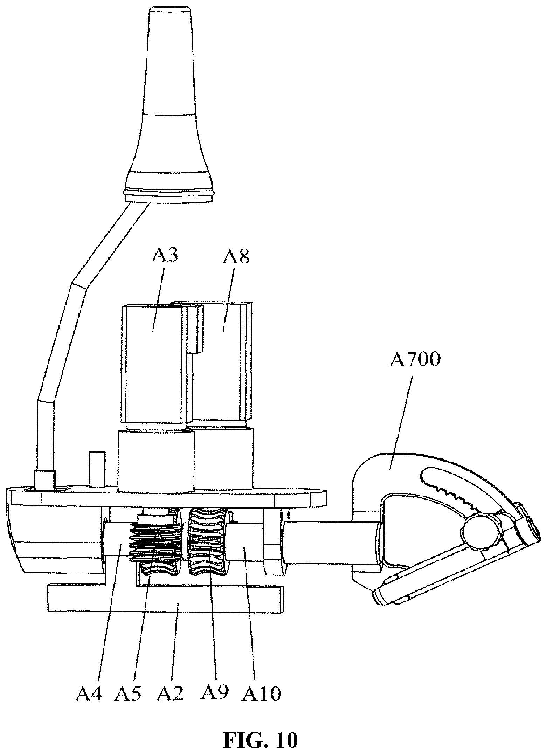

Referring to FIGS. 9 and 10, the ultrasonographic device in this embodiment further includes a second driver A8 and a second transmission mechanism A9. The second driver A8 is disposed in the mounting housing A1, and an output end of the second driver A8 is connected to the second transmission mechanism A9. The second transmission mechanism A9 is connected to a second main shaft A10, and the second main shaft A10 extends out of the avoiding hole on the mounting housing A1 and is connected to the puncture support A700. The second driver A8 is, for example, an electric motor.

The output of the second driver A8 and the output of the first driver A3 are controlled at the same time to respectively drive the puncture support A700 and the ultrasonic transducer A2 to synchronously rotate. That is, the output of the second driver A8 controls the rotation of the puncture support A700, and the output of the first driver A3 controls the rotation of the ultrasonic transducer A2. When the puncture needle A900 bends outside the image plane and cannot be seen in the ultrasound image plane, only the first driver A3 is controlled to start without moving the puncture needle A900, that is, only the ultrasonic transducer A2 is controlled to rotate, so as to change a position of the image plane to search for the puncture needle A900.

Exemplarily, the second transmission mechanism A9 is connected to the output end of the second driver A8 through a gear. The second transmission mechanism A9 includes a worm, a worm gear is coaxially mounted on the second main shaft A10, and the worm drives the worm gear to rotate. In other embodiments, the second driver A8 is drivingly connected to the second transmission mechanism A9 through a belt drive, a chain drive, or the like, and the second transmission mechanism A9 is drivingly connected to the second main shaft A10 through a belt drive, a chain drive, or the like.

In an embodiment, the second main axis A10 may be coaxial with the first main axis A4, so as to ensure that the puncture path on the puncture support A700 is always on the real-time scanning plane A800 of the ultrasonic transducer A2.

In other embodiments, the second driver A8 and the second transmission mechanism A9 may be disposed outside the mounting housing A1, so as to ensure the sealing property of the mounting housing A1 and improve the service life of the ultrasonic transducer A2.

### Embodiment three

The difference between this embodiment and embodiment one is described below.

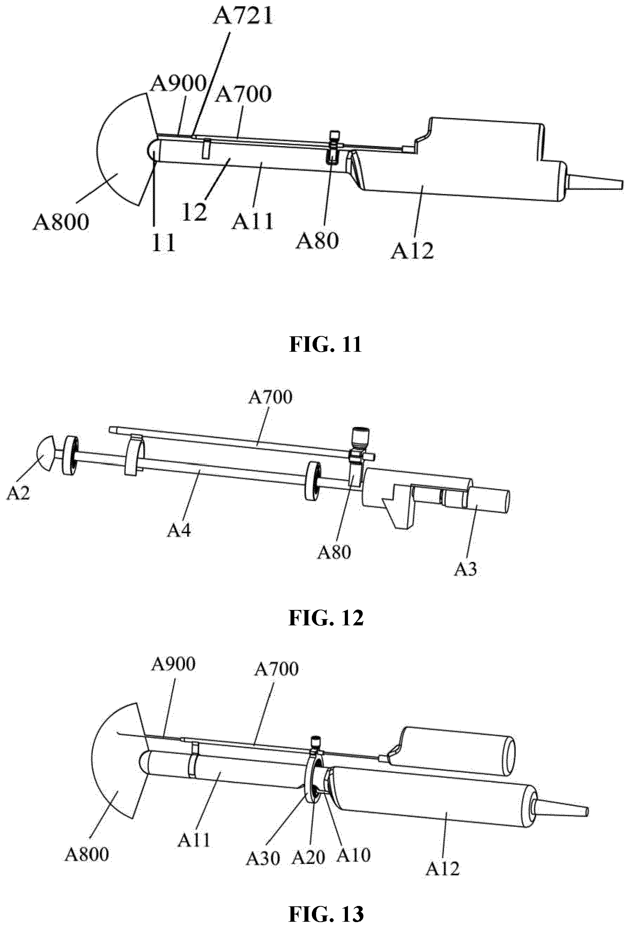

Referring to FIGS. 11 and 12, in the ultrasonographic device in this embodiment, the mounting housing A1 is elongated. The mounting housing A1 includes a probe end housing A11 and a housing A12 communicating with each other. The probe end housing A11 includes a head 11 and an acoustically transparent housing 12 (for example, an acoustically transparent hollow rod). The head 11 is plugged at an end of the acoustically transparent housing 12. The housing A12 communicates with the other end of the acoustically transparent housing 12. Both the ultrasonic transducer A2 and the first main shaft A4 are disposed in the acoustically transparent housing A1 1. The first driver A3 is disposed in the housing A12. An avoiding hole is provided on the acoustically transparent housing 12. The first main shaft A4 extends out of the acoustically transparent housing 12 via the avoiding hole through a connector A80 and is connected to the puncture support A700. In this embodiment, the connector A80 is a connection block A80. The puncture support A700 is also elongated. The axis of the puncture guide hole A721 on the puncture support A700 is parallel to an axis of the first main shaft A4 and an axis of the acoustically transparent housing 12, the axis of the first main shaft A4 penetrates through or is parallel to a centerline of the ultrasound image, and the axis of the puncture guide hole A721 is parallel to a centerline of the real-time scanning plane of the ultrasonic transducer A2. The ultrasonographic device is applicable to cavities (such as the rectum and vagina) for diagnosis and treatment of the colorectum, uterus, follicles, or other sites and organs.

Exemplarily, the acoustically transparent housing 12 can physically isolate the ultrasonic transducer from contact with the outside world and allow ultrasonic signals to pass through. The acoustically transparent housing 12 may be elongated. In different embodiments, different parts may be configured to function as the acoustically transparent housing.

The head 11 in FIG. 11 may function as the acoustically transparent housing.

Exemplarily, the ultrasonic transducer A2 in this embodiment is a convex array ultrasonic transducer A2. The ultrasonic transducer A2 is disposed at an end of the first main shaft A4. The ultrasound transducer A2 rotates around the axis of the first main shaft A4. The centerline of the scanning plane A800 of the ultrasonic transducer A2 is collinear with the axis of the first main shaft A4. The puncture guide hole A721 on the puncture support A700 is parallel to the centerline of the scanning plane A800 of the ultrasonic transducer A2.

### Embodiment four

The difference between this embodiment and embodiments one and three is described below.

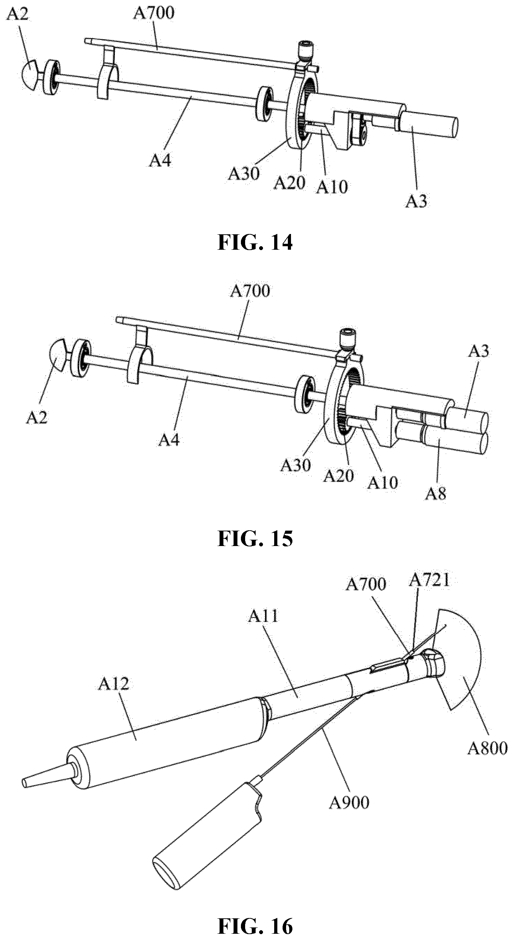

Referring to FIGS. 13 and 14, the ultrasonographic device provided in this embodiment further includes a second main shaft A10. The output end of the first driver A3 is drivingly connected to both the first main shaft A4 and the second main shaft A10.

For example, the output end of the first driver A3 is directly connected to the first main shaft A4. The output end of the first driver A3 is connected to the second main shaft A10 through a belt drive. The second main shaft A10 extends out of the mounting housing A1 and is drivingly connected to the puncture support A700.

An outer gear A20 is coaxially mounted on part of the second main shaft A10 extending out of the mounting housing A1, where the outer gear A20 is engaged with an inner gear A30, and an outer circumferential wall of the inner gear A30 is fixedly connected to the puncture support A700. The second main shaft A10 drives, through the inner gear A30, the outer gear A20 to rotate, and the outer gear A20 further drives the puncture support A700 to rotate.

In this embodiment, an avoiding opening corresponding to the inner gear A30 is provided on the probe end housing A11.

In other embodiments, the dimensions of the inner gear A30 or the probe end housing A11 and related structures may be adjusted, so as to avoid interference between the inner gear A30 and the probe end housing A11. In this case, the probe end housing A11 does not need to have an avoiding opening, so as to improve the sealing property.

In addition, compared with embodiment three in which the connection block A80 extends out of the acoustically transparent housing 12 of the probe end housing A11, in this embodiment, the second main shaft A10 extends out of the mounting housing A1 and is connected to the puncture support A700. In this embodiment, it is more convenient to provide a sealing device such as the sealing ring so that the sealing property is better.

### Embodiment five

The difference between this embodiment and embodiment four is described below.

Referring to FIG. 15, the ultrasonographic device provided in this embodiment further includes a second driver A8 and a second main shaft A10 which are disposed in the housing A12. The second driver A8 is, for example, an electric motor. The second main shaft A10 is coaxially connected to an output shaft of the second driver A8. The second main shaft A10 extends out of the housing A12 and is drivingly connected to the puncture support A700.

For example, an outer gear A20 is coaxially mounted on part of the second main shaft A10 extending out of the mounting housing A1, where the outer gear A20 is engaged with an inner gear A30, and the outer gear A20 is fixedly connected to the puncture support A700. The second main shaft A10 drives, through the inner gear A30, the outer gear A20 to rotate, and the outer gear A20 drives the puncture support A700 to rotate.

The ultrasonic transducer A2 and the puncture support A700 rotate synchronously driven by the first driver A3 and the second driver A8, respectively. When the puncture needle A900 bends outside the image plane and cannot be seen in the image plane, only the first driver A3 is controlled to start without moving the puncture needle A900, that is, only the ultrasonic transducer A2 rotates, so as to change the position of the image plane to search for the puncture needle A900.

### Embodiment six

The difference between this embodiment and embodiment three is described below.

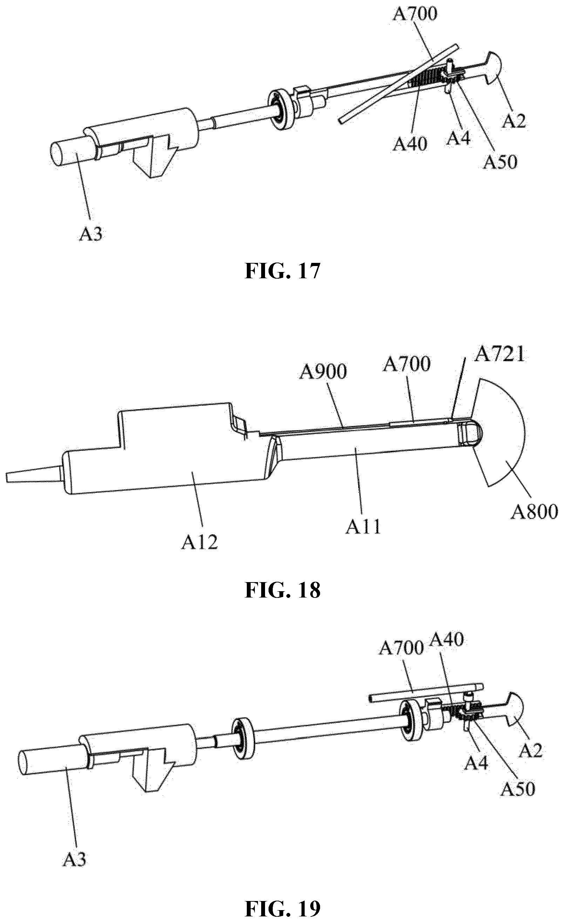

Referring to FIGS. 16 and 17, in the ultrasonographic device provided in this embodiment, in the acoustically transparent housing 12 of the probe end housing A11, the output end of the first driver A3 is connected to a rack A40, and a gear A50 is coaxially mounted on the first main shaft A4. The output end of the first driver A3 drives, through the rack A40, the gear A50 to rotate, the first main shaft A4 rotates coaxially with the gear A50, and the ultrasonic transducer A2 and the puncture support A700 swing around the first main shaft 24. The axis of the first main shaft A4 is separately perpendicular to the output end of the first driver A3 and an axis of the acoustically transparent housing 12, or the axis of the first main shaft A4 is separately at an angle to the output end of the first driver A3 and the axis of the acoustically transparent housing 12. In other embodiments, other drive means such as a lead screw nut may be used between the first driver A3 and the first main shaft A4.

Exemplarily, the ultrasonic transducer A2 is a convex array ultrasonic transducer A2. The centerline of the scanning plane A800 of the ultrasonic transducer A2 is perpendicular to and coplanar with the first main shaft A4. A sweeping region of the ultrasonic transducer A2 in this embodiment is different from a sweeping region of the ultrasonic transducer A2 in embodiment three.

The puncture support A700 is connected to the first main shaft A4 through a connecting rod, and the puncture support A700 and the ultrasonic transducer A2 swing synchronously around the first main shaft A4. The puncture guide hole A721 on the puncture support A700 is at an angle to the centerline of the scanning plane A800 of the ultrasonic transducer A2. The axis of the puncture guide hole A721 on the puncture support A700 is at an angle to the axis of the acoustically transparent housing 12 of the probe end housing A11, so as to adjust an occupied space of the whole ultrasonographic device, so that the ultrasonographic device can be adapted to different surgical operation spaces.

Exemplarily, an overlapping part of the puncture needle A900 and the puncture support A700 is the puncture guide hole A721. The puncture guide hole A721 may be an elongated channel and may have a circular shape, but may also have a shape other than a circular shape. The puncture guide hole A721 allows the puncture needle A900 to penetrate through and can fix a heading direction of the puncture needle A900.

Exemplarily, in FIG. 16, the puncture support A700 is a small circular tube, and a channel of an inner hole of the puncture support A700 is the puncture guide hole A721. In FIG. 16, only one puncture guide hole A721 is provided.

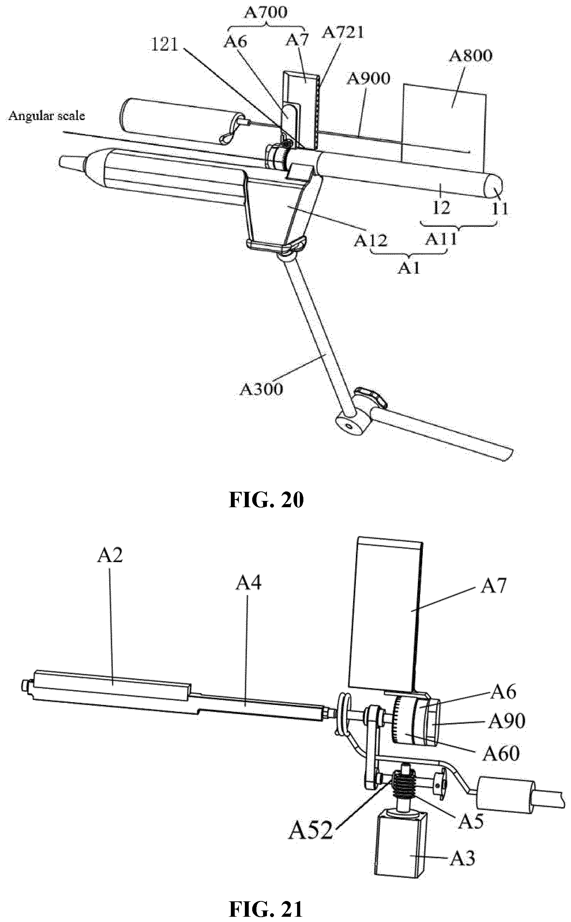

This embodiment differs from embodiment eight in that in FIG. 20, multiple puncture supports A700 are fixedly provided.

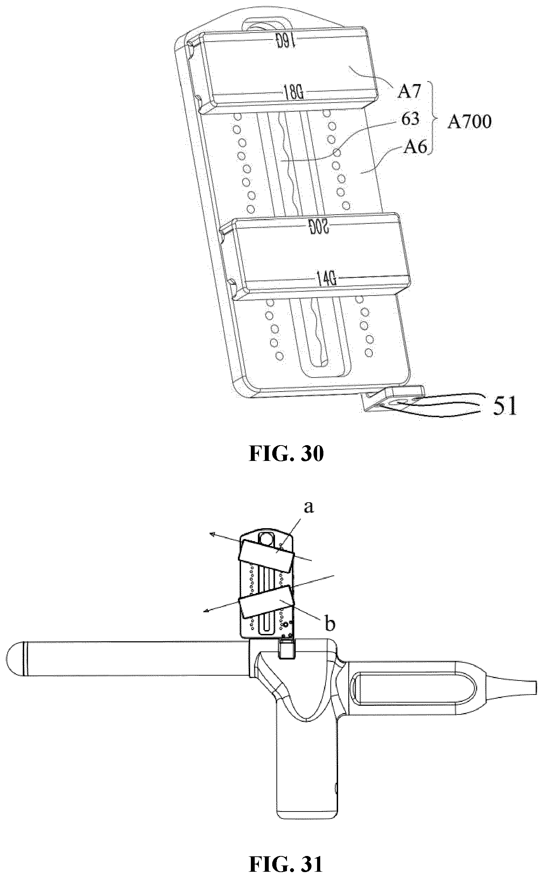

This embodiment differs from embodiment nine in that, in FIG. 30, multiple puncture supports A700 may be provided to adapt to the puncture needles A900 with different diameters. Generally, only one puncture needle A900 is used and selected by a user in a single operation. In addition, the position of the puncture guide hole A721 is configured to be adjustable by the user in position and angle.

### Embodiment seven

The difference between this embodiment and embodiment six is described below.

Referring to FIGS. 18 and 19, an axis of the puncture support A700 and the axis of the puncture guide hole A721 on the puncture support A700 are parallel to the axis of the acoustically transparent housing 12 of the probe end housing A11, so as to adjust the occupied space of the whole ultrasonographic device, so that the ultrasonographic device can be adapted to different surgical operation spaces. The puncture guide hole A721 on the puncture support A700 is parallel to the centerline of the scanning plane A800 of the ultrasonic transducer A2.

### Embodiment eight

Referring to FIGS. 20 to 23, this embodiment provides an ultrasonographic device. The ultrasonographic device includes the ultrasound probe A400 and the puncture support A700. The ultrasound probe A400 includes the probe end housing A11 and the housing A12. The probe end housing A11 is elongated. The probe end housing A11 includes the head 11 and the acoustically transparent housing 12. The head 11 is plugged at an end of the acoustically transparent housing 12. The housing A12 communicates with the other end of the acoustically transparent housing 12. The ultrasonic transducer A2 and the first main shaft A4 are mounted in the acoustically transparent housing 12 of the probe end housing A11. The first driver A3 is disposed in the housing A12. The first driver A3 is, for example, an electric motor. The first driver A3 is drivingly connected to the first main shaft A4 through the first transmission mechanism A5.

Exemplarily, the head 11 in the probe end housing A11 may function as a generic housing which is distinguished from the acoustically transparent housing.

The first transmission mechanism A5 includes the worm gear A52 and a belt drive assembly. The worm gear A52 is connected to a worm at the output end of the first driver A3. The worm gear A52 is connected to the first main shaft A4 through the belt drive assembly.

An end of the first main shaft A4 facing away from the ultrasonic transducer A2 extends out of an end port of the acoustically transparent housing 12 facing away from the head 11, and is fixedly connected to a connecting stub shaft A60 coaxially. The puncture support A700 includes the carrier A6 and the puncture guide member A7. The carrier A6 includes the connecting portion A61 and the carrier body A62. A fastener A90 penetrates through the connecting portion A61 and is connected to the connecting stub shaft A60 to fix the carrier A6 to the connecting stub shaft A60. The fastener A90 is, for example, a screw or bolt. The puncture guide member A7 is movably provided on the carrier A6.

In this embodiment, the acoustically transparent housing 12 of the probe end housing A11 is fixedly connected to the housing A12. The connecting stub shaft A60 is coaxially disposed at the end port of the acoustically transparent housing 12 facing away from the head 11 and fixedly connected to the first main shaft A4. The connecting stub shaft A60 rotates together with the first main shaft A4, that is, the connecting stub shaft A60 is also rotatably connected to the acoustically transparent housing 12. In this embodiment, a scale is provided on an outer circumferential wall of the connecting stub shaft A60, and an indicating structure 121 is provided on an outer wall of an end of the acoustically transparent housing 12 facing away from the head 11. When the connecting stub shaft A60 rotates together with the first main shaft A4, the indicating structure can be aligned with the scale having different angle values, and the indicating structure can indicate an angle of rotation of the first main shaft A4 and the connecting stub shaft A60 relative to the acoustically transparent housing 12, which is convenient for an operator to adjust an angle of rotation of the ultrasonic transducer A2 more finely.

Exemplarily, in FIG. 20, in conjunction with an angular scale, the indicating structure 121 functions as a protractor.

A mating hole is provided on the connecting stub shaft A60, and an end of the first main shaft A4 facing away from the ultrasonic transducer A2 extends out of the acoustically transparent housing 12 and is inserted into the mating hole. An ejector pin penetrates in a radial direction of the connecting stub shaft A60, and the thimble presses the first main shaft A4 into the mating hole. After the thimble is pulled out, the connecting stub shaft A60 is rotated to adjust a mounting position of the carrier A6 on the first main shaft 46 in a circumferential direction, so as to adapt to different diagnostic operation spaces.

In an embodiment, a first positioning groove A601 and a first positioning protrusion A611 are respectively provided on a surface of the connecting stub shaft A60 and a surface of the connecting portion A61 which face each other. When the fastener A90 is screwed to lock the connecting stub shaft A60 and the connecting portion A61, the first positioning protrusion A611 is gradually clamped into the first positioning groove A601, thereby improving the stability of the connection between the carrier A6 and the connecting stub shaft A60, facilitating assembly, and having high repeated assembly accuracy. In other embodiments, positions of the first positioning groove A601 and the first positioning protrusion A611 are interchangeable, that is, the first positioning groove A601 is provided on the connecting portion A61, and the first positioning protrusion A611 is provided on the connecting stub shaft A60.

The carrier body A62 is provided with a connecting hole A622, a connecting post A74 is convexly provided on the puncture guide member A7, and the connecting post A74 penetrates through the connecting hole A622 and is connected to a nut A70, so as to mount the puncture guide member A7 on the carrier body A62. The nut A70 is loosened, and the puncture guide member A7 is driven to rotate relative to the carrier body A62 so that after the connecting post A74 rotates in the connecting hole A622, the nut A70 is tightened again, so as to achieve the rotation of the puncture guide member A7 relative to the carrier body A62.

Multiple puncture guide holes A721 with different dimensions and arranged in parallel are provided on the puncture guide member A7. Each puncture guide hole A721 corresponds to a letter.

In an embodiment, a second positioning protrusion A75 is convexly provided on the puncture guide member A7, multiple second positioning grooves A623 are concavely provided on the carrier body A62, and the second positioning protrusion A75 may be selectively clamped into any of the second positioning grooves A623, so as to control an angle of rotation of the puncture guide member A7.

In this embodiment, three second positioning grooves A623 are provided so that the puncture guide member A7 has three puncture states. The three second positioning grooves A623 are configured to be second positioning groove one A6231, second positioning groove two A6232, and second positioning groove three A6233respectively.

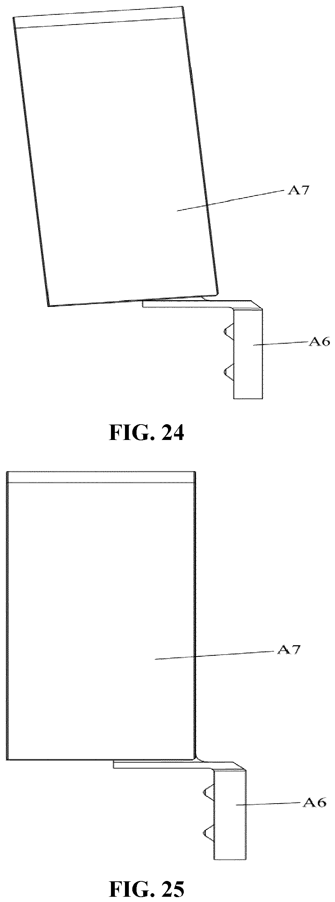

As shown in FIG. 24, when the second positioning protrusion A75 is clamped into the second positioning groove one A6231, the puncture guide hole A721 is at a negative angle to the first main shaft A4, and the head of the puncture needle A900 faces downward, which is a downward oblique puncture state.

As shown in FIG. 25, when the second positioning protrusion A75 is clamped into the second positioning groove two A6232, the puncture guide hole A721 is parallel to the first main shaft A4, and the head of the puncture needle A900 is horizontal, which is a horizontal puncture state.

As shown in FIG. 26, when the second positioning protrusion A75 is clamped into the second positioning groove three A6233, the puncture guide hole A721 is at a positive angle to the first main shaft A4, and the head of the puncture needle A900 faces upward, which is an upward oblique puncture state.

For a detailed description of the puncture states, reference may be made to embodiment nine.

A mounting manner of the puncture guide member A7 on the carrier A6 in this embodiment may be a mounting manner of the puncture guide member A7 on the carrier A6 in embodiment nine. The details are not repeated.

### Embodiment nine

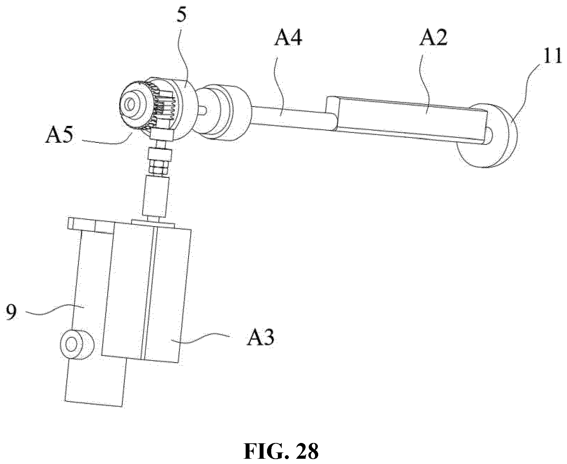

As shown in FIGS. 27 and 28, this embodiment provides an ultrasonographic device. The ultrasonographic device includes the probe end housing A11, the first main shaft A4, the ultrasonic transducer A2, the first driver A3, a mounting ring 5, and the puncture support A700. The probe end housing A11 includes the head 11 and the acoustically transparent housing 12 (for example, a hollow rod). The head 11 is fixedly connected to an end of the acoustically transparent housing 12. The first main shaft A4 rotatably penetrates through the acoustically transparent housing 12 along an axial direction of the first main shaft A4. The ultrasonic transducer A2 is fixedly connected to the first main shaft A4. The ultrasonic transducer A2 is located inside the acoustically transparent housing 12. The ultrasonic transducer A2 is spaced apart from the acoustically transparent housing 12. A movable end of the first driver A3 is drivingly connected to an end of the first main shaft A4 facing away from the head 11. The first driver A3 can drive the first main shaft A4 to drive the ultrasonic transducer A2 to rotate. The first driver A3 in this embodiment is a motor. The mounting ring 5 is fixedly sleeved outside the first main shaft A4. Multiple mounting holes 51 are provided on the mounting ring 5. The puncture support A700 is mounted on the mounting ring 5 through the multiple mounting holes 51.

In the ultrasonographic device provided in this embodiment, the ultrasonic transducer A2 is fixed on the first main shaft A4, and the puncture support A700 is mounted on the mounting ring 5 fixed on the first main shaft A4 so that the puncture support A700 and the ultrasonic transducer A2 can rotate synchronously. In the ultrasonographic device proposed in this embodiment, the ultrasonic transducer A2 and the puncture support A700 can rotate synchronously, thereby ensuring that the puncture path is on a real-time scanning plane of the ultrasound probe, so as to achieve a function of accurate puncture guiding and obtain a more accurate diagnosis and treatment effect.

For example, the ultrasonographic device further includes the first transmission mechanism A5, where an input end of the first transmission mechanism A5 is fixedly connected to the movable end of the first driver A3, and an output end of the first transmission mechanism A5 is fixedly connected to the end of the first main shaft A4 facing away from the head 11. The first transmission mechanism A5 can keep the ultrasonic transducer A2 and the puncture support A700 stably rotating synchronously during the puncturing so that the displacement of the ultrasonic transducer A2 and the puncture support A700 due to the puncture operation is avoided.

In an embodiment, the first transmission mechanism A5 in this embodiment uses a worm gear and worm mechanism with the self-locking function. For the first transmission mechanism A5 without the self-locking function, the introduction of electric motor torque locking or additional electric or manual locking is required.

For example, in FIG. 30, when the puncture support A700 is not used, the puncture support A700 is removed and a dust cover is used to cover the multiple mounting holes 51. At the same time, sealing rings are provided on two sides of the mounting ring 5 to prevent water and dust from entering the inside and affecting the internal structure and function.

Exemplarily, the puncture support A700 includes the carrier A6 and the puncture guide member A7, where the carrier A6 is connected to the mounting ring 5, the puncture guide member A7 is provided on the carrier A6, multiple puncture guide holes A721 with different dimensions are provided on the puncture guide member A7, and a biopsy needle can penetrate through the puncture guide hole A721 and positioned.

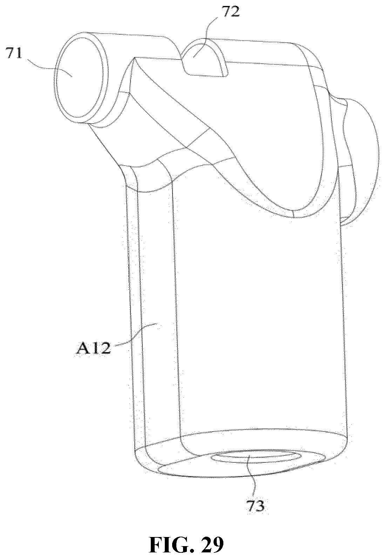

As shown in FIGS. 27 and 29, to protect the internal structure and ensure the aesthetic appearance of the device, the device further includes the housing A12, where a first opening 71 is provided on the housing A12, the housing A12 is connected to an end of the acoustically transparent housing 12 facing away from the head 11 through the first opening 71, the first driver A3, the first transmission mechanism A5, and the mounting ring 5 are disposed inside the housing A12, a second opening 72 is disposed at a position of the mounting ring 5 on the housing A12, the puncture support A700 is capable of being mounted on the mounting ring 5 through the second opening 72, and when driving the first main shaft A4 to rotate, the first driver A3 is capable of driving, through the mounting ring 5, the puncture support A700 to rotate. The ultrasonic transducer A2 and the puncture support A700 fixed on the first main shaft A4 rotate synchronously, thereby ensuring that the biopsy needle penetrating through the puncture support A700 is on a plane of a scanning image of the ultrasound probe. The case where the ultrasound probe cannot monitor the puncture process is avoided.

As shown in FIGS. 27 and 30, an adjustment slot 63 is provided on the carrier A6, and the puncture guide member A7 is movable along an extension direction of the adjustment slot 63. Multiple puncture guide members A7 are provided, and dimensions of the puncture guide holes A721 on the multiple puncture guide members A7 may be different. As shown in FIG. 30, each of 14G, 16G, 18G, 20G, and the like separately corresponds to the puncture guide hole A721 of one dimension.

Exemplarily, the puncture guide member A7 is clamped and mounted in the adjustment slot 63 through a bolt, the puncture guide member A7 is rotatable around the bolt, and three puncture states during rotation are described below.

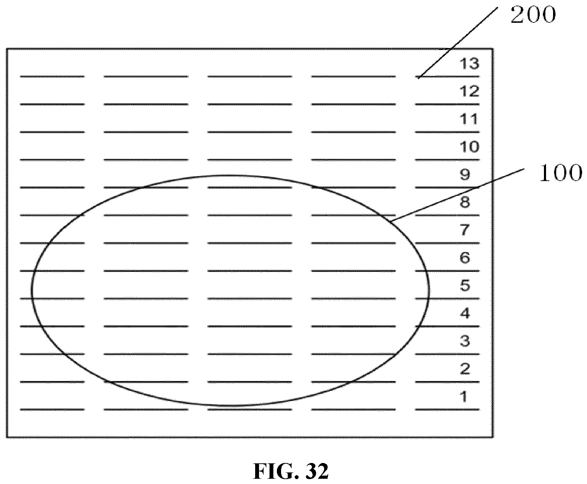

As shown in FIG. 27, a state in which the puncture guide hole A721 is parallel to the first main shaft A4 is the horizontal puncture state. In this case, the position of the puncture guide member A7 is adjusted up and down so that multiple horizontal puncture channels 200 can be obtained. The ultrasonic display is shown in FIG. 32.

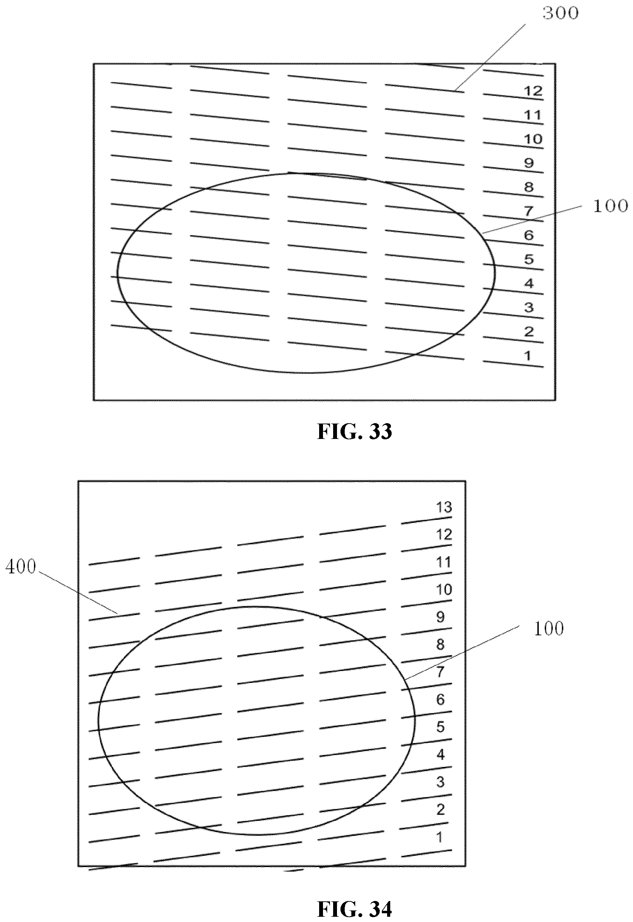

As shown in FIG. 31, the head of the biopsy needle on the puncture guide member A7 labeled "a" faces obliquely upward of the first main shaft A4 and is at a positive angle to the first main shaft A4, which is the upward oblique puncture state. In this case, the position of the puncture guide member A7 is adjusted up and down so that multiple upward oblique puncture channels 300 in parallel can be obtained. The ultrasonic display is shown in FIG. 33.

As shown in FIG. 31, the head of the biopsy needle on the puncture guide member A7 labeled "b" faces obliquely downward of the first main shaft A4 and is at a negative angle to the first main shaft A4, which is the downward oblique puncture state. In this case, the position of the puncture guide member A7 is adjusted in up and down so that multiple downward oblique puncture channels 400 in parallel can be obtained. The ultrasonic display is shown in FIG. 34.

As shown in FIGS. 32 to 34, a target object 100 is located within the coverage of multiple puncture channels, so as to aim at the target object 100. The number (for example, 1 to 13) on each puncture channel is the number of the puncture channel and used for calibrating a position of the target object 100. The biopsy needle may perform puncture inspection along the puncture channels.

To facilitate gripping, as shown in FIG. 27, the ultrasonographic device further includes a grip portion 8 disposed at the end of the acoustically transparent housing 12 facing away from the head 11.

In an embodiment, as shown in FIGS. 28 and 29, a mounting seat 9 is provided on a side of the first driver A3, the mounting seat 9 is connected to the first driver A3, a fixing hole is disposed at an end of the mounting seat 9 facing away from the first main shaft A4, and the fixing hole is used for mounting and fixing the ultrasonographic device. Correspondingly, a third opening 73 is disposed at a corresponding position on the housing A12, and the fixture A200 mounts and fixes the ultrasonographic device through the third opening 73.

A device other than the human hand is required to achieve stable fixation of the system when the system is operating. An external device such as the fixture A200 may be used for stabilization.

## Claims

1. An ultrasonographic device, comprising:
a mounting housing (A1);
an ultrasonic transducer (A2) rotatably disposed in an inner cavity of the mounting housing (A1) and spaced apart from the mounting housing (A1); and
a puncture support (A700) provided on an outer side of the mounting housing (A1), wherein a puncture guide hole (A721) through which a puncture needle (A900) penetrates is provided on the puncture support (A700), and the puncture support (A700) is capable of rotating synchronously with the ultrasonic transducer (A2) so that an axis of the puncture guide hole (A721) is coplanar with a real-time scanning plane (A800) of the ultrasonic transducer (A2).

2. The ultrasonographic device of claim 1, further comprising a first driver (A3) and a first main shaft (A4) which are disposed in the mounting housing (A1), wherein an output end of the first driver (A3) is connected to the first main shaft (A4), the first main shaft (A4) is connected to the ultrasonic transducer (A2), and the first main shaft (A4) extends out of the mounting housing (A1) and is connected to the puncture support (A700).

3. The ultrasonographic device of claim 2, wherein the mounting housing (A1) comprises a probe end housing (A11), wherein the probe end housing (A11) comprises a head (11) and an acoustically transparent housing (12), wherein the head (11) is plugged at an end of the acoustically transparent housing (12), the first main shaft (A4) is disposed in the acoustically transparent housing (12), the ultrasonic transducer (A2) is connected to the first main shaft (A4), and the first main shaft (A4) extends out of the acoustically transparent housing (12) through a connector (A80) and is connected to the puncture support (A700).

4. The ultrasonographic device of claim 3, wherein the first main shaft (A4) is disposed coaxially with the acoustically transparent housing (12), and the ultrasonic transducer (A2) is rotatable about an axis of the first main shaft (A4).

5. The ultrasonographic device of claim 3, wherein an axis of the first main shaft (A4) is arranged at an angle to an axis of the acoustically transparent housing (12), and the ultrasonic transducer (A2) is swingable around the axis of the first main shaft (A4).

6. The ultrasonographic device of claim 5, wherein a rack (A40) extending along an axial direction is provided in the acoustically transparent housing (12), the first main shaft (A4) is coaxially connected to a gear (A50) engaged with the rack (A40), and the rack (A40) is movable driven by the first driver (A3) to drive the gear (A50) to rotate.

7. The ultrasonographic device of claim 1, further comprising a first driver (A3), a first main shaft (A4), and a second main shaft (A10) which are disposed in the mounting housing (A1), wherein an output end of the first driver (A3) is separately connected to the first main shaft (A4) and the second main shaft (A10), the first main shaft (A4) is connected to the ultrasonic transducer (A2), and the second main shaft (A10) extends out of the mounting housing (A1) and is connected to the puncture support (A700).

8. The ultrasonographic device of claim 1, further comprising a first driver (A3) and a second driver (A8), wherein an output end of the first driver (A3) is connected to the ultrasonic transducer (A2), an output end of the second driver (A8) is connected to the puncture support (A700), and the ultrasonic transducer (A2) and the puncture support (A700) rotate synchronously driven by the first driver (A3) and the second driver (A8), respectively.

9. The ultrasonographic device of claim 8, wherein the first driver (A3) and the second driver (A8) are both disposed in the mounting housing (A1), a second main shaft (A10) is connected to the output end of the second driver (A8), and the second main shaft (A10) extends out of the mounting housing (A1) and is connected to the puncture support (A700).

10. The ultrasonographic device of claim 7 or 9, wherein part of the second main shaft (A10) extending out of the mounting housing (A1) is coaxially connected to an outer gear (A20), wherein the outer gear (A20) is engaged with an inner gear (A30), and an outer circumferential wall of the outer gear (A20) is connected to the puncture support (A700).

11. The ultrasonographic device of claim 3, wherein the puncture support (A700) comprises a carrier (A6), wherein the carrier (A6) comprises a connecting portion (A61) and is fixed on a connecting stub shaft (A60); and
a first positioning protrusion (A611) is provided on one of the connecting stub shaft (A60) or the connecting portion (A61), and a first positioning groove (A601) is provided on the other one of the connecting stub shaft (A60) or the connecting portion (A61), wherein the first positioning protrusion (A611) is configured to be clamped into the first positioning groove (A601).

12. The ultrasonographic device of claim 1, wherein the mounting housing (A1) comprises a probe end housing (A11), wherein the probe end housing (A11) comprises a head (11) and an acoustically transparent housing (12), wherein the head (11) is fixedly connected to an end of the acoustically transparent housing (12); and wherein the ultrasonographic device further comprises:
a first main shaft (A4) rotatably disposed the acoustically transparent housing (12) along an axial direction of the first main shaft (A4), wherein the ultrasonic transducer (A2) is fixedly connected to the first main shaft (A4);
a first driver (A3), wherein a movable end of the first driver (A3) is drivingly connected to an end of the first main shaft (A4) facing away from the head (11), and the first driver (A3) is capable of driving the first main shaft (A4) to drive the ultrasonic transducer (A2) to rotate; and
a mounting ring (5) fixedly sleeved outside the first main shaft (A4), wherein a plurality of mounting holes (51) are provided on the mounting ring (5), and the puncture support (A700) is mounted onto the mounting ring (5) through the plurality of mounting holes (51).

13. The ultrasonographic device of claim 12, further comprising a first transmission mechanism (A5), wherein an input end of the first transmission mechanism (A5) is fixedly connected to the movable end of the first driver (A3), and an output end of the first transmission mechanism (A5) is fixedly connected to the end of the first main shaft (A4) facing away from the head (11).

14. The ultrasonographic device of claim 13, further comprising a housing (A12), wherein a first opening (71) is provided on the housing (A12), the housing (A12) is connected to an end of the acoustically transparent housing (12) facing away from the head (11) through the first opening (71), the first driver (A3), the first transmission mechanism (A5), and the mounting ring (5) are disposed inside the housing (A12), a second opening (72) is disposed on the housing (A12) at a position of the mounting ring (5), the puncture support (A700) is capable of being mounted on the mounting ring (5) through the second opening (72), and when driving the first main shaft (A4) to rotate, the first driver (A3) is capable of driving the puncture support (A700) to rotate.

15. The ultrasonographic device of claim 12, wherein the puncture support (A700) comprises a carrier (A6) and a puncture guide member (A7), wherein an adjustment slot (63) is provided on the carrier (A6), the puncture guide member (A7) is movable along an extension direction of the adjustment slot (63), the puncture guide member (A7) is clamped and mounted in the adjustment slot (63) through a bolt, the puncture guide member (A7) is rotatable around the bolt, and three puncture states during rotation comprises:
a horizontal puncture state when the puncture guide hole (A721) is parallel to the first main shaft (A4);
an upward oblique puncture state when the puncture guide hole (A721) is at a positive angle to the first main shaft (A4); and
a downward oblique puncture state when the puncture guide hole (A721) is at a negative angle to the first main shaft (A4).
